# EUROPEAN PATENT APPLICATION

(11) **EP 4 560 644 A1**
(43) Date of publication of application: **28.05.2025**
(21) Application number: 23315438.4
(22) Date of filing: 24.11.2023
(51) Int. Cl.: G16H 30/40, G16H 50/20, G16H 50/30

(54) **METHODS FOR TRAINING AND USING A MACHINE LEARNING MODEL FOR PREDICTING A LIKELIHOOD OF AN EVENT OCCURRING IN A PATIENT HAVING A MALIGNANT TUMOUR**

(71) Applicant: University of Zurich, 8006 Zürich (CH)
(72) Inventor: Fremond, Sarah Corinne Margot, 2333ZA Leiden (NL); Bosse, Tjalling, 2333 ZA Leiden (NL); Horeweg, Nanda, 2333 ZA Leiden (NL); Koelzer, Viktor Hendrik, 8006 Zürich (CH)
(74) Representative: HGF

(57) **Abstract**

Methods for training and using a machine learning model for predicting a likelihood of an event occurring in a patient having a malignant tumour are provided. The method of using the machine learning model includes inputting a pathology image of a portion of a malignant tumour of a patient and one or more categorical risk factors associated with the malignant tumour or the patient. The method also includes extracting, from the pathology image, one or more features relating to one or more properties of the malignant tumour. The method further comprises determining a predicted likelihood of an event occurring in the patient based on the extracted features and the one or more categorical risk factors. The method additionally includes outputting the predicted likelihood.

## Description

### TECHNICAL FIELD

This invention relates to a method for predicting a likelihood of an event occurring in a patient having a malignant tumour. The invention additionally relates to training a machine learning model for performing the method, a trained model obtained from the training method, a computer-readable storage medium, and a system configured to perform a method according to the invention.

### BACKGROUND

When a patient is found to have a malignant tumour, it is desirable to develop a most appropriate personalised treatment plan for that patient. Using prognostic factors relating to the malignant tumour in order to accurately predict a likelihood of one or more clinical endpoints occurring in the patient is vital for developing this treatment plan.

However, the optimal use of prognostic factors in the clinic remains a challenge. Current analysis of malignant tumours is typically performed through a visual analysis of the tumour, or a portion thereof, by a clinician. It is difficult to consistently obtain high-quality outcomes from this type of analysis because identifying all relevant histopathological variables and assigning relative weightings to each of the identified variables in combination with other prognostic variables of the patients are subject to high interobserver variability and not fully understood at this time. These challenges naturally lead to less accurate predictions regarding a prognosis for the patient.

It is in this context that the invention is devised.

### BRIEF SUMMARY

In one aspect, a computer-implemented method for predicting a likelihood of an event occurring in a patient having a malignant tumour is provided. The method is performed by a trained model. The method includes inputting a pathology image of a portion of a malignant tumour of a patient and one or more categorical risk factors associated with the malignant tumour or the patient. The method also includes extracting, from the pathology image, one or more features relating to one or more properties of the malignant tumour. The method further comprises determining a predicted likelihood of an event occurring in the patient based on the extracted features and the one or more categorical risk factors. The method additionally includes outputting the predicted likelihood.

Using a trained machine learning model to determine a predicted likelihood of an event occurring provides a consistent method for obtaining a prediction. This method is less prone to factors such as human error or interpretational variability, which can impact both the consistency and the accuracy of the resulting predictions. Predictions obtained by the trained model are therefore more reliable than those obtained by a clinician. This allows a clinician to make more informed decisions regarding a patient's care, resulting in better outcomes for the patient.

This trained model advantageously requires only an image of a portion of the malignant tumour and at least one categorical risk factor associated with the malignant tumour as inputs. These inputs are objective and do not rely upon a clinician's prior subjective interpretation or analysis. For example, human interpretations of histopathologic features in oncology images are known to be susceptible to variability both between clinicians and by the same clinician over time. However, the image itself is a definitive input which is neither dependent on nor influenced by a clinician's perspective of the image. The overall quality (consistency and accuracy) of the output predictions is improved by relying only on objective inputs rather than potentially biased inputs.

Furthermore, the inputs required by the model are cost-efficient inputs which are typically readily available for patients having a malignant tumour. Aside from scanning the tumour slide to obtain the image, it is not necessary to perform additional laboratory work to derive the inputs. This reduces costs and potential time delays. The method of the present invention is therefore relatively inexpensive and quick.

The use of both the image of the malignant tumour and the one or more categorical risk factors as inputs improves the accuracy of the predictions compared to models in which only one of these inputs are used. Moreover, the processing of these two types of inputs together by the trained model further improves the accuracy of the predictions. That is, the model does not merely obtain a prediction based on a first input (for example, the image of the malignant tumour) and then fine-tune the obtained prediction based on the second input (for example, the one or more categorical risk factors), but jointly processes the two types of inputs from start-to-finish to determine the prediction. Considering the inputs together, rather than separately, further improves the accuracy of the predictions generated by the model.

Where the one or more categorical risk factors comprises at least two categorical risk factors, the trained model jointly processes the image of the tumour and each of the two or more categorical risk factors together. In other words, the model does not combine the plurality of categorical risk factors into a single variable. The model also does not process the image of the tumour together with a first categorical risk factor, and then subsequently introduce the second (or more) categorical risk factor into the processing. The image of the tumour, the first categorical risk factor, the second categorical risk factor, and any additional categorical risk factors input into the model are jointly processed to generate the predicted likelihood.

Optionally, the trained model predicts one or more further categorical risk factors based on the extracted one or more features. The trained model may determine the predicted likelihood of the event based on the features extracted from the image, the one or more input categorical risk factors, and the one or more predicted categorical risk factors.

The accuracy of the predicted likelihood is further enhanced by additionally considering the categorical risk factors predicted by the model when determining the prediction because the additional information leads to improved likelihood predictions. The predicted categorical risk factors are processed jointly with the image of the malignant tumour and the one or more categorical risk factors that are input into the model, in the same manner as described above. This advantageously further improves the accuracy of the likelihood prediction.

Using the trained model to predict categorical risk factors from the input image, rather than directly inputting pre-determined categorical risk factors, can further improve the cost-effectiveness of the method and the overall time to obtain the prediction. This is because using the model to predict further categorical risk factors can be cheaper and faster than performing an analysis in a laboratory to determine the actual further categorical risk factors. In addition, the trained model can be used in hospitals and other facilities which do not have the necessary equipment for determining actual values for the further risk factors.

Optionally, the one or more predicted categorical risk factors comprises at least a predicted molecular feature of the malignant tumour. The predicted molecular feature may be a predicted molecular classification of the malignant tumour.

Using the predicted molecular feature of the malignant tumour in addition to the input image and the input categorical risk factors further improves the accuracy of the predicted likelihood determined by the model. This also enables a clinician to verify the veracity of the outputs of the trained model. For example, if the predicted molecular feature is additionally output by the model, a clinician may compare the predicted molecular feature with an actual molecular feature obtained via an alternative means (such as a laboratory analysis or professional judgement) so that the accuracy of at least a portion of the model's working can be independently confirmed for at least a portion of the patients. This leads to clinicians having an improved confidence in the performance of the model.

The molecular classification is a known prognostic factor relating to some types of malignant tumours, such as endometrial cancers.. When the predicted molecular feature is a well-established feature such as the molecular classification, this enables the veracity of the trained model's predictions to be more easily measured.

Optionally, the trained model treats each categorical risk factor as learnable categorical information. The trained model also forms an embedding for each of the categorical risk factors.

These categorical risk factors include both the input categorical risk factors and the predicted categorical risk factors generated by the trained model.

Treating the categorical risk factors as learnable categorical information and forming embeddings (or continuous vectors) for each of the categorical risk factors improves the ability of the model to learn the relationships between the categorical risk factors and the features extracted from the pathology images.

Continuous vectors are typically used to represent data that is continuous in nature, such as image features processed by deep learning models. Categorical data is typically processed using different methods designed for discrete data to keep the discrete nature of the data. However, continuous vectors using embedding methods are typically used for word embeddings in natural language processing to represent categorical data in a continuous way in lower dimensional space. However, the predictions output by the trained model were surprisingly found to be more accurate when the categorical risk factors were transformed in continuous vectors using embedding methods in combination with higher dimension mapping.

Using embeddings or continuous vectors preserves the spatial relationships between the variables within the categorical risk factors for the trained task. Transforming categorical data with embeddings creates a space where the proximities of continuous vectors representing the categorical data are determined according to a similarity of their impacts on the trained task. That is, the spatial relationships between the continuous vectors representing categorical variables reflect the relationships between the variables and their influence on determining the predicted likelihood. This improves the ability of the model to recognise, for example, that a 70 year old patient is more likely to have a similar prognosis to a 71 year old patient than a 20 year old patient, if other factors are the same. If the spatial relationships between the age variables were not preserved, the model would not necessarily as readily recognise the relationships between the comparative age gaps and their likely prognoses. Whilst the spatial relationships between variables such as an age may be clear to a clinician, the relationships between non-ordinal variables (such as different genetic mutations) are much less apparent.

Optionally, the trained model determines the predicted likelihood by forming an image-level embedding for the input pathology image. The trained model further combines each of the categorical risk factor embeddings and the image-level embedding to form a single fused embedding.

Forming a single fused embedding ensures that all of the inputs are used when determining the predicted likelihood, so no input is overlooked. The model therefore uses all of the information available to it when determining the predicted likelihood, so the prediction is more reliable.

Optionally, the trained model forms the single fused embedding by applying a gating-based attention mechanism to each of the categorical risk factor embeddings and the image-level embedding. The trained model then performs an outer product to each of the embeddings based on a result of the gating-based attention mechanism.

This enables the model to weight the various categorical risk factors and the image features according to the importance of the variable to the end result. That is, the different variables are not necessarily given equal consideration when determining the predicted likelihood. Instead, the variables that have more significance are weighted to have more influence on the predicted likelihood than variables which have a lesser impact on the outcome. For example, if it is found that the image features have a greater bearing on a prognosis of the patient than the age of the patient, then the model will assign more attention to the image-level embedding than the age factor embedding. Attributing a weight to each of the embeddings according to an importance of the embedding factor in determining the end result therefore improves the accuracy of the prediction provided by the model.

It is very difficult for a clinician to accurately weigh a number of different variables, especially variables of different types, when forming predictions relating to a patient's treatment or prognosis. The use of a machine learning model to perform this step therefore improves the accuracy of the predictions, which leads to more evidence-based decision making by the clinician.

Optionally, the one or more input categorical risk factors comprises prognostic metadata or a personal characteristic of the patient. The prognostic metadata may comprise a stage category of the malignant tumour. The personal characteristic may comprise one or more of an age, ethnicity, body mass index, gender or underlying health condition of the patient.

Prognostic metadata, such as the stage category of the tumour, is well-established prognostic information which is commonly obtained for patients with malignant tumours. The prognostic metadata also typically requires little or no interpretation from a clinician. Risk factors relating to personal characteristics of a patient are also readily available and non-subjective. Using well-established, non-subjective variables enables widespread implementation of the invention since highly-specialised facilities or knowledge are not required to derive the inputs. The predictions obtained from the model are also more consistent using this method.

Optionally, the predicted likelihood of an event is a predicted time to a clinical endpoint. Further optionally, the clinical endpoint is a recurrence of the malignant tumour, a progression of the malignant tumour or a death of the patient.

Being aware of a predicted time to a clinical endpoint (or a predicted likelihood of experiencing the event within a given time period, or a predicted likelihood of not experiencing the event within a given time period) allows clinicians to make more informed decisions regarding a patient's care. For example, a clinician may decide whether a patient should undergo adjuvant treatment for a malignant tumour based on a result of the prediction. If the predicted recurrence-free probability within the next 5 years is 20%, the clinician may decide that the patient would benefit from adjuvant treatment to minimise the risk of recurrence. On the other hand, if the predicted recurrence free probability within the next 5 years is 90%, the clinician may determine that adjuvant treatment is not an appropriate course of treatment for the patient at this time. Being able to make clinical decisions based on a predicted time to a clinical endpoint reduces under and over-treatment of patients. This allows resources to be better targeted to patients who are more likely to benefit, whilst also ensuring that patients do not unnecessarily undergo difficult, invasive and expensive treatments.

The clinical endpoints of a malignant tumour recurrence, a malignant tumour progression, and a lifespan of a patient are important endpoints for a clinician to consider when determining an appropriate personalised treatment plan for the patient. For example, having accurate estimations of a time until one or more of these clinical endpoints may enable a clinician to select one or more of surgery, medication, therapy, adjuvant therapy or palliative care as part of the treatment plan for the patient.

In a second aspect, a computer-implemented method for training a machine learning model to predict a likelihood of an event occurring in a patient having a malignant tumour is provided. The method includes receiving a labelled training set for the machine learning model. The labelled training set includes, for each of a plurality of patients, a pathology image of a malignant tumour of the patient, one or more categorical risk factors associated with the malignant tumour or the patient, and data relating to the patient's experience with an event. The method also comprises training the machine learning model, using the labelled training set, to extract one or more features from each pathology image in the training set. The method further comprises training the machine learning model to relate the one or more extracted features and the one or more external categorical risk factors with the event.

This method results in a trained machine learning model which can consistently and accurately determine a predicted likelihood of an event occurring in a patient having a malignant tumour. The training of the machine learning model does not require any prognostic information which is not well-established and readily obtainable for patients having malignant tumours.

Optionally, training the machine learning model further comprises additionally relating a predicted categorical risk factor to the event. The risk factor is predicted by the machine learning model based on the received pathology image for each of the plurality of patients. The predicted categorical risk factor may be a predicted molecular feature of the malignant tumour. The predicted molecular feature may be a predicted molecular classification of the malignant tumour.

Optionally, the data relating to the patient's experience with the event includes a time to the event if the patient has experienced the event or a time to a most recent assessment for the event if the patient has not experienced the event.

This data allows the machine learning model to learn the relationships between the input pathology image features and the one or more categorical risk factors and either an amount of time it took for a patient to experience the event or, where a patient has not experienced the event, a time to a most recent assessment for the event.

For patients who have not experienced the event, the amount of time that has expired without the patient experiencing the event also provides valuable information. This data allows the machine learning model to learn which characteristics from the input pathology images and the categorical risk factors may be associated with longer waiting times for the event or a non-occurrence of the event within a particular time period.

Optionally, the event comprises a clinical end point and the predicted likelihood of an occurrence of the event comprises a predicted time to the clinical endpoint. The clinical endpoint may be a recurrence of the malignant tumour, a progression of the malignant tumour or a death of the patient.

In a third aspect, there is provided a trained model obtained from any of the methods set out in relation to the second aspect.

In a fourth aspect, there is provided a computer-readable storage device including computer-executable instructions, which when executed by a computing system, are capable of causing the computing system to perform the method as described in relation to the first aspect or the second aspect.

In a fifth aspect, there is provided a system configured to perform the method according to the first aspect or the second aspect.

The advantages set out in relation to any aspect apply equally to corresponding features of other aspects. Descriptions have not been repeated for conciseness.

### BRIEF DESCRIPTION OF THE SEVERAL VIEWS OF THE DRAWINGS

One or more embodiments of the invention are shown schematically, by way of example only, in the accompanying drawings, in which:
FIG. 1 shows a flowchart for using a trained deep-learning model in accordance with embodiments of the invention.
FIG. 2 shows a flowchart for using a trained model in accordance with embodiments of the invention.
FIG. 3 shows a flowchart for training a deep learning model in accordance with embodiments of the invention.
FIG. 4 shows a vision transformer model in accordance with embodiments of the invention.
FIG. 5 shows a deep learning model in accordance with embodiments of the invention.
FIG. 6 shows results obtained from a deep learning model according to an embodiment of the invention.

### DETAILED DESCRIPTION

Hereinafter, embodiments of the disclosure are described with reference to the accompanying drawings. However, it should be appreciated that the disclosure is not limited to the embodiments, and all changes and/or equivalents or replacements thereto also belong to the scope of the disclosure. The same or similar reference denotations may be used to refer to the same or similar elements throughout the specification and the drawings.

As used herein, the terms "have," "may have," "include," or "may include" a feature (e.g., a number, function, operation, or a component such as a part) indicate the existence of the feature and do not exclude the existence of other features.

As used herein, the terms "A or B," "at least one of A and/or B," or "one or more of A and/or B" may include all possible combinations of A and B. For example, "A or B," "at least one of A and B," "at least one of A or B" may indicate all of (1) including at least one A, (2) including at least one B, or (3) including at least one A and at least one B.

The terms as used herein are provided merely to describe some embodiments thereof, but not to limit the scope of other embodiments of the disclosure. It is to be understood that the singular forms "a," "'an," and "the" include plural references unless the context clearly dictates otherwise. All terms including technical and scientific terms used herein have the same meaning as commonly understood by one of ordinary skill in the art to which the embodiments of the disclosure belong. It will be further understood that terms, such as those defined in commonly used dictionaries, should be interpreted as having a meaning that is consistent with their meaning in the context of the relevant art and will not be interpreted in an idealized or overly formal sense unless expressly so defined herein. In some cases, the terms defined herein may be interpreted to exclude embodiments of the disclosure.

An example deep learning (DL) model has been developed to provide information indicative of a risk of a clinical event occurring in a patient. This model has been tested using real data from a plurality of patients and has obtained favourable results. The example model has been trained to predict a distant recurrence-free probability over a time period in endometrial cancer patients. The skilled person would understand that DL models for other types of cancers could be obtained using equivalent processes. The skilled person would also recognise that DL models for other types of clinical events could also be obtained using equivalent processes. The example DL model is a Histopathology-based Endometrial Cancer Tailored treatment and distant recurrence Outcome Risk prediction model, known as HECTOR.

Endometrial cancer is a type of cancer originating in the tissues of the uterus. It is the most common gynaecological malignancy in the Western world, with rising incidence globally due to aging and obesity. Primary treatment for endometrial cancer is hysterectomy. Although the majority of post-surgical patients have a good prognosis without adjuvant treatment, 10-20% of patients will develop distant recurrence (DR). DR is associated with a poor five-year survival rate.

Recurrence is a term used to describe the return of a cancer in a patient after the cancer has been previously treated. The recurrence may be local, regional, or distant. DR occurs when a cancer spreads to organs or tissues far from the original cancer source. Chemotherapy is currently the only available adjuvant treatment for reducing the likelihood of developing DR. However, chemotherapy is a toxic treatment, and patients with a low risk of experiencing DR receive little benefit from this treatment. It is therefore desirable to accurately predict patients at risk of developing DR so that adjuvant treatment can be better targeted.

In brief, HECTOR is a two-step deep learning model: a state-of-the-art vision transformer for multi-resolution patch-level self-supervised learning representation, and a novel multimodal three-arm architecture to predict time-to-distant recurrence. The three-arm architecture fuses prognostic information from a pathology image of a portion of the malignant tumour, the image-based molecular class as predicted by a further DL model for predicting the molecular class of the malignant tumour directly from the image, and the stage I-III of the tumour. The DL model for predicting the molecular class may be a separately trained model that is integrated into HECTOR. Attention-based multiple instance learning and embedding layers which map the discrete image-based molecular class and stage are combined to a higher-dimensional continuous vector space. Each arm's importance is controlled using a gating-based attention mechanism and the attention-weighted embeddings are then fused to capture interactions between the three arms.

The invention will be described further in this specification in both general terms and with reference to the specific example of HECTOR. The skilled person would readily understand that HECTOR is a specific example of how the invention may be implemented, and is by no means the only way in which a DL model for predicting a risk of a clinical event may be achieved. The invention is therefore not limited to a specific cancer, a specific type of clinical event or the specific implementation demonstrated by HECTOR.

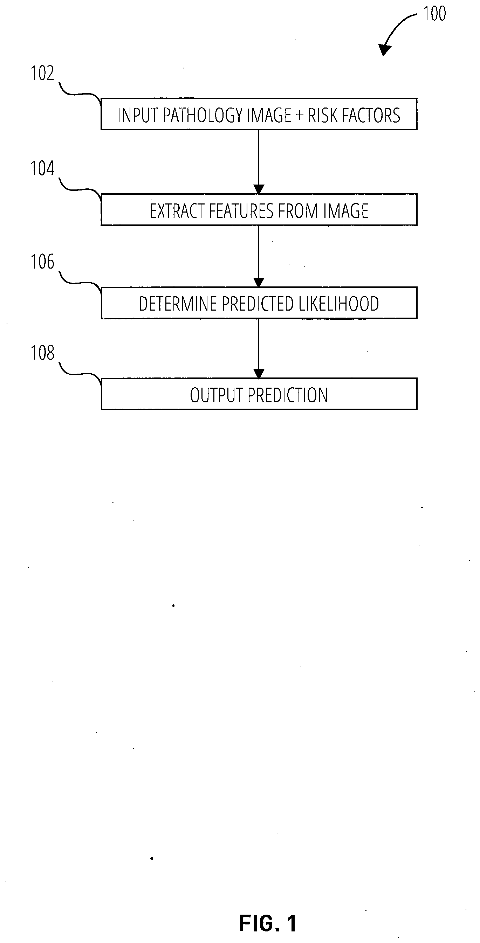

Figure 1 illustrates a method 100 for using a trained machine learning model to predict a likelihood of an event occurring in a patient having a malignant tumour. The machine learning model is a deep learning model. That is, it has a minimum of four layers - an input layer, an output layer, and at least two hidden layers. The input layer receives the input data and passes the input data to the first hidden layer. The output layer is the last layer in the deep learning model, and produces the output of the model. The hidden layers are the layers which perform the majority of the computational processing to learn the patterns in the input data and associate these patterns with an output. In general, increasing the number of hidden layers in the deep learning model increases the complexity of the patterns that the model can learn. The machine learning model may use a convolutional neural network (CNN).

In step 102, a pathology image showing at least a portion of the malignant tumour of the patient is input into the machine learning model. In addition, one or more categorical risk factors relating to the malignant tumour or the patient are input into the model.

The malignant tumour, which hereon may simply be referred to as a tumour, may be any tumour which can be viewed on a pathology image. The tumour may be a solid tumour, such as a carcinoma, a sarcoma, or a melanoma. The tumour may relate to a gynaecological cancer. The tumour may relate to an endometrial cancer. For the avoidance of doubt, these are merely examples to assist with the understanding of the invention. The invention is not intended to be limited to any of these examples. That is, tumour or cancer types which have not been mentioned are not to be assumed as excluded from the invention.

The pathology image is a digital image of a tissue sample taken from the tumour. The pathology image may comprise a scanned histology image where the tissue sample from the tumour is placed on a slide and scanned at a high resolution using a microscope. The pathology image may be termed a whole slide image (WSI). The tissue sample may be stained to distinguish different tissue structures and cell types within the sample. For example, the image may be a haematoxylin and eosin (H&E) stained image.

The one or more categorical risk factors relate to the tumour or the patient in any combination. The categorical risk factors are any non-visual features which could influence a predicted prognosis of the patient. For example, categorical risk factors relating to the tumour may include prognostic metadata such as a stage category of the tumour. As another example, categorical risk factors relating to the tumour may include determined molecular properties of the tumour, such as a molecular classification of the tumour.

Categorical risk factors relating to the patient may include personal characteristics including (but not limited to) the age, gender, ethnicity, family medical history, body mass index, underlying health conditions, medication taken, or lifestyle factors (such as smoking status, alcohol consumption, recreational drug use) of the patient.

In step 104, the machine learning model extracts one or more features from the input pathology image. The one or more features may be extracted by the machine learning model itself, or by a second trained model nested within the machine learning model. Any method suitable for extracting complex visual information from the pathology image, such as inputting the pathology image into a vision transformer model, may be used to extract the features.

The features extracted from the pathology image may comprise one or more of morphological features relating to the shape and/or appearance of the cells and tissues of the tumour, textural features relating to the arrangement of the cells and tissues of the tumour, and contextual features relating to the relationships between the cells and tissues of the tumour.

In step 106, the machine learning model determines a predicted likelihood of a clinical event occurring in a patient having a malignant tumour. The model uses the features extracted from the pathology image and the one or more input categorical risk factors to generate the prediction. The extracted features and the one or more input categorical risk factors may be provided to a neural network for determining the predicted likelihood.

The predicted likelihood may comprise a predicted likelihood of experiencing the event within a given time period, or a predicted likelihood of not experiencing the event within a given time period. The predicted likelihood may comprise a hazard function for a time point. The predicted likelihood may comprise a survival function for a time point. The predicted likelihood may comprise a predicted time to the clinical event occurring in the patient. The clinical event may be a clinical endpoint such as a recurrence of the malignant tumour, a progression of the malignant tumour or a death of the patient, or an absence or non-occurrence of these endpoints. The recurrence of the malignant tumour may be a local, regional or distant recurrence. The progression of the malignant tumour may be a progression of the tumour to a different stage category, or other measurement relating to the size or a spread of the tumour.

The machine learning model may determine the predicted likelihood using both the extracted features and the one or more categorical risk factors jointly. That is, the model may not form a prediction based on either the extracted features or the one or more categorical risk factors, and then refine the prediction using the other input. Instead, the machine learning model performs an end-to-end operation using both types of input together.

In step 108, the model outputs the determined predicted likelihood of the clinical event occurring. The predicted likelihood may be output as a predicted time to the clinical event, or a predicted hazard function for the time point. The predicted likelihood may be output by assigning a risk status to the patient. For example, the model may output an indication of whether the patient has a high, low or medium risk of experiencing the clinical event. The model may output an indication of whether the patient has a high, low or medium risk of experiencing the clinical event within a certain timeframe. The timeframe may be one year, five years, ten years, or any suitable timeframe appropriate for the type of clinical event.

For example, if the clinical event is a recurrence of the malignant tumour, the model may output a prediction showing that the patient is likely to develop recurrence in three years.. The model may output a prediction indicating that the patient has a high risk of experiencing recurrence within the next five years. The model may output the prediction using a combination of these examples so that the clinician has a variety of information at their disposal.

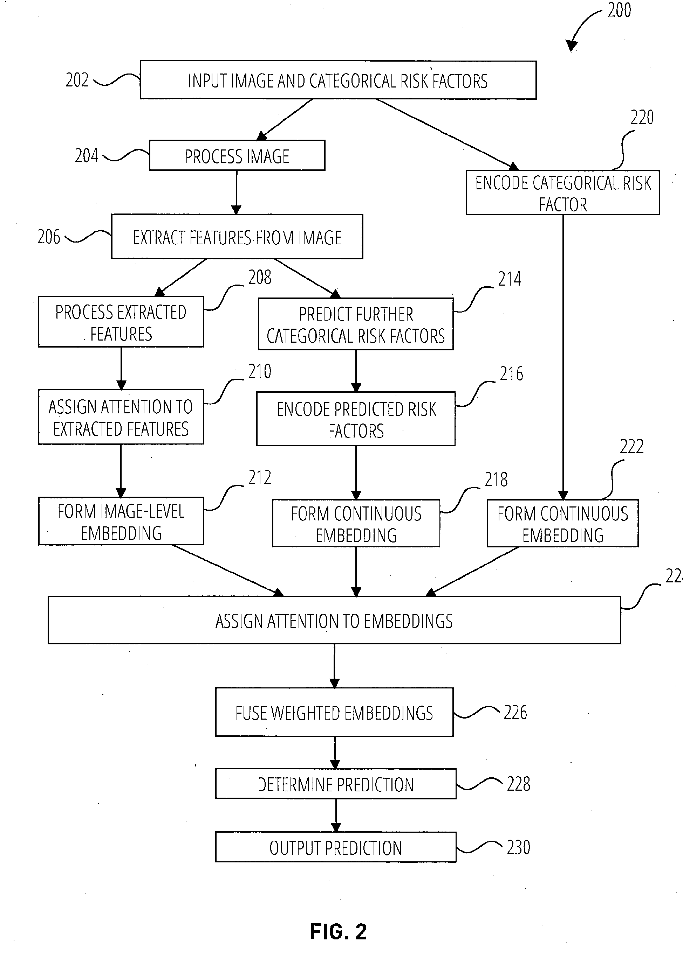

Figure 2 illustrates another method 200 for using a trained machine learning model to predict a likelihood of an event occurring in a patient having a malignant tumour. Where steps in this figure are the same as those described above in relation to Figure 1, detailed descriptions will not be repeated.

In step 202, a pathology image and one or more categorical risk factors are input into the trained machine learning model.

In step 204, the input pathology image is processed. The image may be processed by the machine learning model itself or by a separate trained model nested within the machine learning model. The separate model may be a vision transformer or CNN model, wherein the vision transformer model may be trained to additionally extract features from the image.

The pathology image may be processed to segment the image. In segmenting, the background is removed from the image to leave just the tissues and cells of the tumour. There are many different segmentation techniques which may be used. For example, the segmentation may be performed using Otsu thresholding. ,

The pathology image may be further processed to patch the image. In patching, the image is broken down or divided into a plurality of smaller regions, known as patches, where each patch shows a portion of the image. There are many different techniques for dividing an image into patches. Non-overlapping patching may be used such that each portion of the pathology image is only present in a single patch. This prevents some tissue or cell regions being analysed multiple times and potentially skewing the end predictions. As another example, overlapping patches having different fields of view may be used. The patches may be of any appropriate size whereby the patches are small enough for efficient processing but large enough to provide meaningful information. The patches may be resized for efficient processing or to improve the extraction of features from the patches.

In step 206, one or more features are extracted from the pathology image. That is, one or more features relating to the malignant tumour may be extracted from one or more of the patches formed from the pathology image. The extracted features may be referred to as patch-level features. The features may be extracted by the machine learning model itself or by a separate trained model nested within the machine learning model. The separate model may be a vision transformer or CNN model. The vision transformer model may be the same model used to process the image in step 204. In step 208, the extracted features are processed in order to reduce the number of patches. The patch-level features, which may simply be referred to as patch features, may be spatially and semantically averaged. That is, morphological information in the WSI may be spatially and semantically compressed by averaging the highly correlated features of nearby patches.

In step 210, attention scores are computed for each of the extracted patch features. Where step 208 has been performed, attention scores are computed for the processed extracted patch features.

Before computing attention scores, the extracted patch-level features from step 206 (or the mean patch-level features from step 208, where step 208 has been performed) may be compressed using a number of connected layers. These compressed patch-level features are then used for the computation of the attention scores. For example, the patch-level features may be compressed by three fully connected layers. This compression reduces the size of the extracted patch-level features. The number of layers used in the compression indicates the number of times that the size of the features are reduced. Processing the extracted features in this manner has been found to improve the performance of the machine learning model. That is, predictions made by the machine learning model are found to be more accurate when this additional processing step is performed compared to when this step is not performed.

Assigning attention scores to patch features indicates how much weight should be applied to the patch features when determining the overall prediction. That is, some types of patch features may be more important when determining a likelihood of a clinical event occurring than other patch features. Rather than treating each of the extracted features with equal consideration, patch features which are known to have a greater influence are assigned more attention. This improves the accuracy of the predictions by ensuring that small yet significant patch features are not outweighed by larger insignificant patch features. Attention scores may be assigned to each of the extracted patch features using any appropriate method. For example, a gating-based attention mechanism may be used.

In step 212, an image-level embedding forms as a result of applying the computed attention score to the patch-level features. The computed attention scores and the patch-level features are each represented as matrices. An outer product is applied between the matrix of attention scores and the matrix of patch features, in a process known as attention pooling, to obtain a weighted image-level embedding..

In step 214, the features extracted from the pathology image are used to predict one or more further categorical risk factors relating to the malignant tumour. The predicted categorical risk factors may be any variables which can be derived from the pathology image, such as a predicted molecular feature. The predicted molecular feature may be a predicted molecular classification of the malignant tumour.

The predicted categorical risk factors may be obtained by the machine learning model itself, or by inputting the extracted features into a third trained model nested within the machine learning model. The third trained model may be a model designed for predicting molecular features from pathology images.

In step 216, the predicted categorical risk factors are encoded as vectors. Where a plurality of categorical risk factors are predicted, each predicted risk factor is processed separately so that a vector is encoded for each risk factor. For example, the vector may be a one-hot vector.

In step 218, the encoded vectors form an embedding. A novel method of transforming each predicted categorical risk factor to a higher-dimensional vector space may be performed. A learnable embedding layer may be applied to the encoded categorical risk factor variables to transform the categorical information into a continuous vector (or embedding). This results in the discrete categorical risk factor variables being represented in a continuous vector space. Transforming the categorical risk factor information to a higher-dimensional vector space allows the model to represent more complex relationships between the categorical variables. Having a better understanding of the relationships between variables enables the machine learning model to make more accurate predictions. A fully connected layer may be applied to the resulting embedding to reduce the size of the resulting embedding.

As a modification to steps 214 to 218, when the features extracted from the pathology image are used to predict one or more further categorical risk factors relating to the malignant tumour in step 214, an intermediate layer prior to the output layer which outputs the predicted risk factor may be used . The intermediate layer may still contain the information relating to the predicted risk factor, but is already in the form of an embedding. It is therefore not necessary to additionally perform the encoding of step 216, or the transforming into a continuous vector space of step 218.

Steps 220 and 222 relate to the input categorical risk factors. These steps are applied individually to each categorical risk factor input into the machine learning model. For simplicity, steps 220 and 222 will be described in relation to a single categorical risk factor. The skilled person would readily understand that, where multiple categorical risk factors are input into the machine learning model, these steps can be applied to all of the input categorical risk factors.

In step 220, the categorical risk factor is encoded as a one-hot vector. This may be achieved in the same way as discussed in relation to step 216.

In step 222, the encoded vector is used to form a embedding. This may be achieved in the same way as discussed in relation to step 218.

In step 224, an attention mechanism is applied to each of the embeddings generated in steps 212, 218 and 222. This controls the importance of each embedding relative to the other embeddings for the trained task. The attention mechanism may be a gating-based attention mechanism. The attention mechanism may be a gating-based attention mechanism with bilinear product. The gating-based attention mechanism improves the performance of the machine learning model by controlling the importance of each information through the deep learning model so that more attention is applied to the embeddings which have a greater influence for the trained task.

In step 226, the weighted embeddings formed in 224 are fused to form a single embedding. The weighted embeddings may be fused using any suitable fusing method. For example, a number (for example, one) may be appended to each of the weighted embeddings in order to capture all of the interactions between the weighted embeddings, and to retain uni-modal embeddings. The appended weighted embeddings may then be fused using the Kronecker product.

in step 228, the fused embedding is processed to generate a predicted likelihood of a clinical event occurring in the patient. The fused embedding may be processed by reducing the embedding using fully connected layers. The fused embedding may be reduced using two fully connected layers of different sizes. This reduces the dimensionality of the fused embedding and results in a fused embedding with a smaller size.

The final fused embedding has an output size which is the same as the number of discrete time intervals for the prediction. The prediction may relate to a hazard function, a survival function, or a risk score indicating a likelihood of the event occurring. The value of each output is indicative of the likelihood of the clinical event occurring within the associated time interval.

In step 230, the generated prediction is output. That is, one or more of the outputs of the final fully connected are provided to the user of the machine learning model.

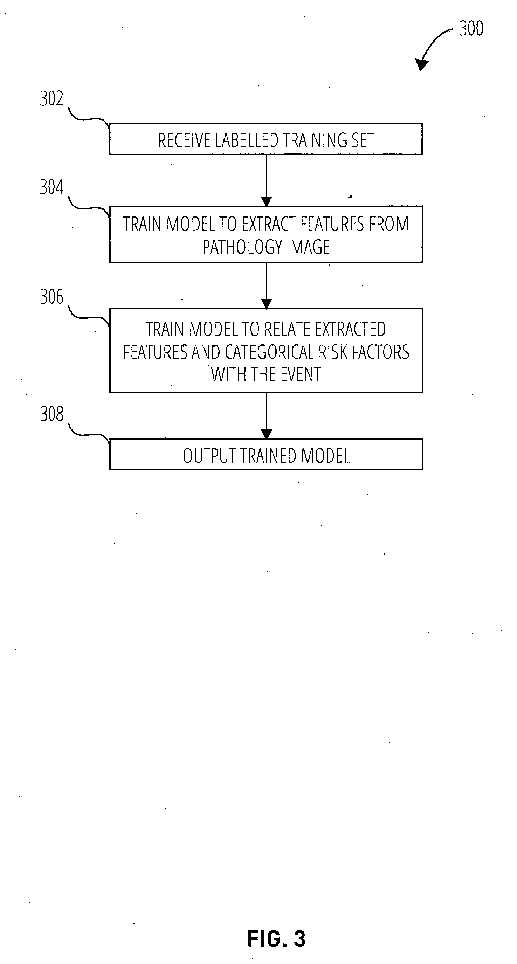

Figure 3 shows a method 300 for training a machine learning model to predict a likelihood of an event occurring in a patient having a malignant tumour.

In step 302, the machine learning model receives a labelled training set. The labelled training set comprises data relating to a plurality of patients who have had a malignant tumour. For each of the plurality of patients, the labelled training set may contain a pathology image of the malignant tumour, one or more categorical risk factors associated with the tumour or the patient, and data relating to the patient's experience with the event.

Where the patient has experienced the event, the data relating to the patient's experience includes an amount of time taken for the patient to experience the event. The amount of time may be a duration from an initial starting point up to a time that the event was first detected or determined in the patient. The initial starting point may be, for example, a first diagnosis of the malignant tumour, a removal of the malignant tumour, or a curing of the malignant tumour.

Where the patient has not yet experienced the event at the time that the training data is taken from the patient, the data relating to the patient's experience may include an amount of time to an end point. The end point may be a most recent assessment for the event. That is, the amount of time may be a duration from a starting point such as a first diagnosis of the malignant tumour, a removal of the malignant tumour, or a curing of the malignant tumour up to an end point, such as a time of a most recent clinical assessment or clinical test result of the patient.

The event may be a clinical endpoint, and the predicted likelihood of an occurrence of the event may be a predicted time to the clinical endpoint. The clinical endpoint may be a recurrence of the malignant tumour, a progression of the malignant tumour or a death of the patient. The clinical endpoint may be an absence of the aforementioned events.

In step 304, the machine learning model is trained to extract features from the received pathology images. This training may be performed by a second machine learning model nested within the first machine learning model. The second machine learning model may be a vision transformer model which is trained to extract features from the pathology images.

In step 306, the machine learning model is trained to relate the extracted features and the one or more categorical risk factors with the event. This training may be performed using a machine learning model which is different to the second machine learning model of step 304. The machine learning model may relate the extracted features and the one or more categorical risk factors with the event by transforming the extracted features into image-level embeddings and each of the categorical risk factors into embeddings. The image-level and categorical risk factor embeddings may be weighted using an attention mechanism and then fused into a single embedding. The fused embedding may then be compared with the data relating to the patient's experience with the event to determine how accurately the fused embedding maps onto the experience data. The machine learning model may then adjust the weightings of each of the variables to try to obtain a fused embedding which more closely matches the experience data across the plurality of patients in the labelled dataset.

The machine learning model may also be trained to predict one or more categorical risk factors from the input pathology image, and to additionally relate the predicted categorical risk factors to the event. The predicted categorical risk factor may be a predicted molecular feature of the malignant tumour, such as a predicted molecular classification.

In step 308, the trained machine learning model is output.

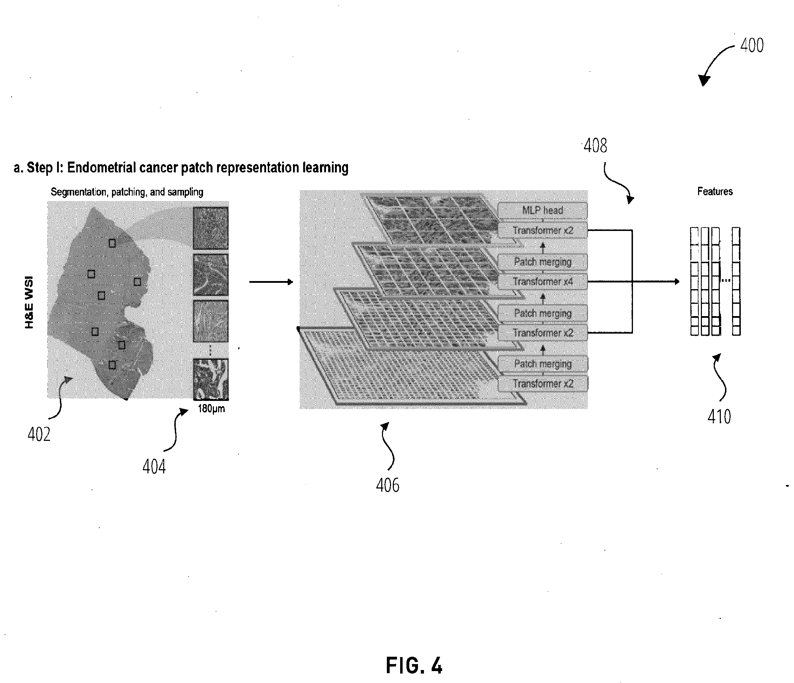

Figure 4 shows an example 400 of how a vision transformer may be used or trained to extract features from a pathology image of a malignant tumour, using HECTOR as the specific example. The vision transformer used is a multi-stage vision transformer, EsVIT, originally developed by Chunyuan Li et al [Chunyuan Li and Jianwei Yang and Pengchuan Zhang and Mei Gao and Bin Xiao and Xiyang Dai and Lu Yuan and Jianfeng Gao. Efficient Self-supervised Vision Transformers for Representation Learning, 2021], but adapted to meet the requirements of HECTOR.

When training the vision transformer, tissue is segmented from the H&E whole slide image 402 of endometrial cancer and subsequently patched 404 at 180µm. The multi-stage vision transformer, EsVIT, is trained with self-supervised learning by randomly sampling the patches obtained from WSIs of 1,862 patients, excluding any patients of the internal and external test sets. The sampled patches are provided to the vision transformer 406, wherein the last eight transformer blocks 408 of the vision transformer 406 are used for extracting patch features 410.

WSI segmentation is performed with Otsu thresholding and non-overlapping patching undertaken at 180µm and resized to 256x256 pixels. On average, this field of view generates a bag of 10,185 squared patches per WSI.

When the trained vision transformer is used by HECTOR, the same method as shown in the example 400 is followed, with some amendments. Rather than randomly sampling patches from the WSI, all of the patches are fed into the trained vision transformer 406. That is, the entire H&E image is patched using non-overlapping patches of size 180µm.

The modifications made to EsVIT from the original transformer set out in the forementioned publication from Chunyuan Li et al are now set out in detail. Advancements in self-supervised learning were followed by adopting vision transformer-based deep learning models that are capable of learning fine-grained patch-level representation at multi-resolutions. Specifically, the initial proposed four-stage Swin transformer-based architecture of EsVIT was modified to fit the present use case in terms of capturing cell-level to gland-level information and meeting our computational resources. The stage-one patch size was doubled to eight pixels to reduce sequence length and enclose cell views. The stages two to four kept the two-factor feature map merging rate, and input images were resized to 256x256 pixels instead of 224 to avoid indivisible patch size at stage 4. Finally, the number of stacked transformers in stage three was reduced from six to four and the rest was kept to two. The first embedding dimension stayed unchanged to 96 and the number of attention heads by stage was kept unchanged, i.e. 3, 6, 12 and 24.

A dataset of 3,702,447 patches was curated by randomly extracting up to 2,000 patches at 180µm resized to 256x256 pixels from the 1,862 WSIs appointed for self-supervised learning. Thereafter, the modified EsVIT was trained on three GPUs Nvidia RTX 8000 with a batch size of 128 for 100 epochs with a window of 14 as it encourages long term dependencies between patches. So-called view and region-level prediction DINO heads with no weight normalization and layers frozen at first epoch were used for performance improvement, and with the default output dimension of 65,536. The authors' recommendations with a smaller batch size were followed by increasing the momentum teacher to 0.9996 and starting with the initial teacher temperature of 0.04. The teacher temperature was adjusted half way through training from 0.04 to 0.02 for further loss decrease. We optimized with AdamW and default Pytorch parameters, and kept default optimization routines of the learning rate (linear warm up for ten epochs followed by cosine scheduler to 1×10⁻⁶) and the weight decay (cosine scheduler from 0.04 to 0.4). No further changes within data augmentation were applied from the original publication. Pytorch (version 1.8.1) was used.

After the training was completed, 180µm patch-level features were extracted from the attention heads of the stacked transformers at each stage. For our downstream task, we found an amelioration by extracting the last eight blocks as compared to the default last four mentioned in the publication of Chunyuan Li et al, yielding feature vectors of size 3,456.

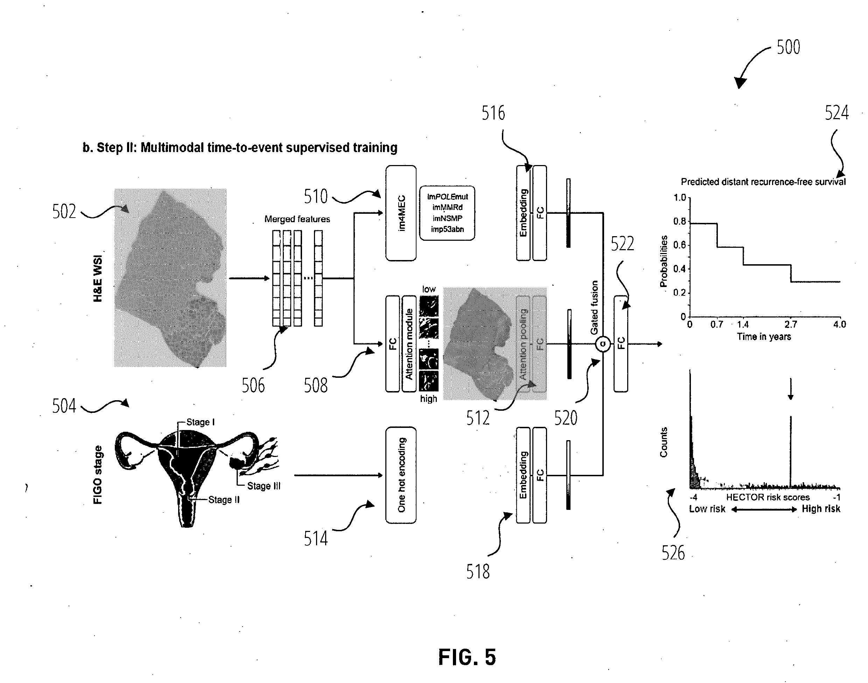

Figure 5 shows an example 500 of how the trained model may be used or trained, using HECTOR as the example.

HECTOR takes a pathology image 502 comprising a H&E WSI of a portion of a malignant tumour from an endometrial cancer (EC) patient and the FIGO 2009 stage I-III category 504 as inputs. The FIGO stage category is a well-established grading system for endometrial tumours, and is used for measuring an extent of an abnormality of the cells in the body. EsVIT is used to extract patch features from the pathology image 502, and the extracted patch features are then spatially and semantically averaged. This reduces the number of patch features.

The reduced number of patch features 506 are passed into both an H&E-based survival deep learning model 508, and im4MEC 510 (with all layers frozen). The model im4MEC was re-trained on each training set in the five-fold cross validation routine with the same training parameters to ensure there was no patient-level overlap.

The deep learning model im4MEC is nested within HECTOR and is a previously trained model for predicting the molecular class from H&E WSI as image-based (im)POLEmut, imMMRd, imNSMP or imp53abn. The molecular class determined by im4MEC is then encoded as a vector. Passing the processed patch features 506 to im4MEC is an optional feature, but has been shown to improve the accuracy of the predictions output by HECTOR since the image-based molecular classes are predictive of prognosis.

The H&E-based survival deep learning model 508 uses three fully connected layers to reduce the dimension of the processed extracted features. An attention mechanism is then used to weight the importance of the extracted features. The extracted features having the highest weights are then used to form a fully connected embedding 512.

In more detail, the H&E-based survival deep learning model (also known as at attention-based multiple instance learning model, AttnMIL model) takes as its input the bag of 180µm patch-level features of size 3,456 extracted from EsVIT where the number of features per bag varies. Given a batch size of one, the time scale is discretized with four intervals based on the quartiles of the distribution of uncensored patients and the last layer is a fully connected layer with four neurons.

Ablation studies have concluded that the attention-based multiple instance learning model outperformed spatial context-aware architectures including graph attention network and transformers with the H&E modality while preserving the least computational complexity with the same loss.

Within the AttnMIL model, we have found a performance increase by adding another WSI preprocessing step: WSI morphological information is spatially and semantically compressed by averaging highly correlated nearby patch-level features using a L2 norm threshold of three patches and a cosine similarity of 0.8. This results in reducing the bag of features from 10,185 patches on average to 1,723 at 180µm.

Each mean patch-level feature is compressed by three fully connected layers gradually down to 512. The attention module computes attention scores on latent features reduced to 256 before pooling, resulting in a slide-level embedding of size 512.

The FIGO stage category is encoded as a vector using one hot encoding 514. Both the encoded FIGO stage category and the encoded image-based molecular class are fed through embedding layers 516, 518.

A gating-based attention mechanism 520 is applied on the resulting three embeddings 512, 516 and 518, followed by a Kronecker product for fusion. This forms the fused embedding 522 from which the predicted likelihoods are obtained. The negative log-likelihood is used to predict the distant recurrence-free survival function over discretized time 524. Risk scores 526 were defined as the integrated predicted survival probabilities.

In more detail, to fuse prognostic information of the categorical stage I-III variable and the image-based four molecular classes derived from the H&E-based predictions of im4MEC to the H&E slide-level embedding, we perform a novel approach of first encoding each categorical information to higher-dimensional vector space with a learnable embedding of size 16 followed by Elu activation function and one fully connected layer of size 8. Next, we apply onto the three resulting embeddings a gating-based attention mechanism with bilinear product to weight the importance of each modality. To capture all interactions and retain uni-modal embeddings, 1 is appended to the attention-weighted embeddings, and these embeddings are then fused using the Kronecker product. The final multimodal embedding is further reduced with two fully connected layers of size 256 and 128 respectively before the survival categorical head which is a fully connected layer where output size is the number of discrete time intervals. Each fully connected layer of the architecture is followed by a Dropout of 0.25 and ReLU activation function. The multimodal survival model was trained for 24 epochs with an initial learning rate of 3×10⁻⁵ decayed by 10 at epoch 2, 5 and 15. The Adam optimizer was used with default parameters and a weight decay of 1×10⁻⁵. Deep learning models were implemented with Pytorch (version 1.10.0).

The acronyms in the example 500 are: MLP = multilayer perceptron; POLEmut *= POLE* mutated; MMRd = mismatch repair deficient; p53abn = p53 abnormal; NSMP = No specific molecular profile; im = image-based; FC = fully connected layer.

Substantially the same steps as shown in example 500 may be used when training HECTOR. When training, data indicative of a time taken for a patient to experience distant recurrence was also input into the model. The predicted distant recurrence-free survival 524 and the risk scores 526 obtained using the steps shown in example 500 were then compared with the input data relating to the time taken for a patient to experience distant recurrence, and differences between the input time data and the output predictions 524 and 526 were calculated. The various weightings applied to the embedding layers were then adjusted based on the calculated differences.

### The Datasets

This study used a H&E WSI dataset of 1,912 EC patients with complete long-term follow-up and clinicopathological data from seven clinical studies including those of the PORTEC-1, -2, and -3 randomized trials to develop and validate a multimodal deep learning model (HECTOR) for predicting distant recurrence-free probabilities from post-surgical stage I-III EC patients.

For supervised training and performance testing purposes, patients with both one representative H&E WSI and distant recurrence outcome data were included from the PORTEC-1/-2/-3 randomized trials and four additional clinical studies. Given the ground-truth data is time to distant recurrence and censorship status, patients with FIGO stage IV and who underwent chemotherapy were removed as this treatment influences the target variable. This yielded 1,912 patients of which one external test set was retained (n = 151 from the complete Leiden cohort), a 20% randomly held-out internal test set (n = 353) and the remaining 80% for training with a five-fold cross-validation routine (n = 1,408). This training dataset was however enriched with dropped WSIs of stage IV patients or those with missing outcome for training with self-supervised learning (n = 1,862).

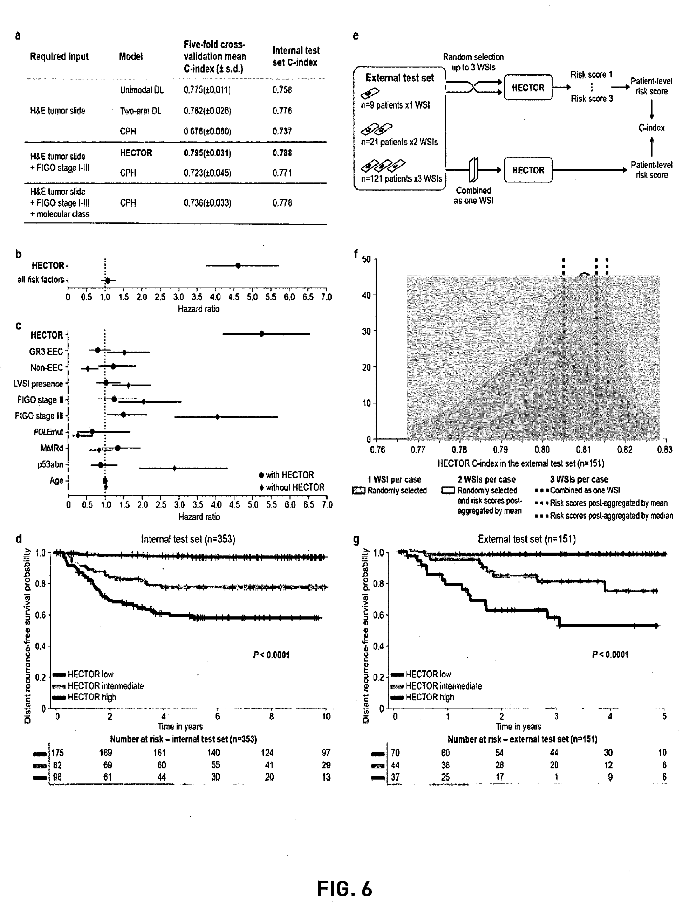

Figure 6 shows graphs detailing the performance of HECTOR. These graphs are explained below.

Graph a shows a comparison of HECTOR performance using the C-index with alternative deep learning-based uni and multimodal model architectures and Cox Proportional Hazard models. Graph b shows a comparison of prognostic values between HECTOR and clinicopathological and molecular risk factors combined into one risk score in a multivariable analysis. Graph c shows a residual prognostic value of all established clinicopathological and molecular risk factors when using HECTOR predicted risk scores in a multivariable analysis. Graph d shows a 10-year distant recurrence-free probabilities analysis using the Kaplan-Meier method by HECTOR risk groups in the internal test set and log-rank test P values. The initial analysis considered all follow-up times, but the graph shows only the results for the first ten years. Graph e shows experiments conducted in the external test set with the input of multiple WSIs. Graph f shows a C-index of HECTOR in the external test set using one to three WSIs per patient. Graph g shows a five-year distant recurrence-free probabilities analysis using the Kaplan-Meier method by HECTOR risk groups when using up to three WSIs (post-aggregated by median) in the external test set and log-rank test P values. G3 = grade 3. EEC = endometrioid endometrial cancer; POLEmut = *POLE* mutated; MMRd = mismatch repair deficient; p53abn = p53 abnormal; LVSI = lymphovascular space invasion. TCGA-UCEC = The Cancer Genome Atlas Uterine Corpus Endometrial Cancer. EBRT = External beam radiotherapy, VBT = Vaginal brachytherapy. MIL = Multiple Instance learning. LVSI = lymphovascular space invasion; s.d. = standard deviation; H&E = hematoxylin-and-eosin; WSI = whole slide image.

During the ablation studies, HECTOR performance was evaluated by computing the mean C-index (and standard deviation) in the five-fold cross-validation (n=1,408 patients). HECTOR demonstrated a mean C-index of 0.795±0.031 on five-fold cross-validation. Notably, the addition of the image-based molecular class arm as predicted by im4MEC boosted performance from 0.775±0.031 to 0.782±0.026 with no need of extra input data, and adding the tumor stage (FIGO I-III) further improved the C-index to 0.795±0.031 (Fig. 6, graph a). We also found that attention-based multiple instance learning outperforms spatially-aware architectures like graph attention networks or transformers using the H&E WSI modality.

On the unseen internal test set (n = 353 patients), HECTOR obtained a C-index of 0.788.

Univariable analysis, using Cox proportional hazard model with continuous HECTOR risk score as the independent variable and time to distant recurrence as the dependent variable, showed strong prognostic value of HECTOR risk score as a continuous variable (hazard ratio (HR) = 4.92; 95% confidence interval (Cl): 4.20-5.76; P < 0.005).

To aid clinical interpretation, we defined categorical HECTOR risk groups at quartiles of the continuous risk scores in the training set, combining groups from the first two quartiles as these had very similar clinical outcomes (distant recurrence-free probabilities of 0.981 and 0.958 respectively; Supplementary Fig. 1). We then tested the prognostic value of these groups in the internal test set. 10-year distant recurrence-free probabilities for HECTOR low (n = 175), intermediate (n = 82) and high (*n* = 96) risk groups were 0.970, 0.777 and 0.581 respectively (log-rank P < 0.001).

We compared DL-based risk scores (that is the one-, two-arm and HECTOR model) with the current standards for EC prognostication comprising clinicopathological factors and the molecular EC classification on the five-fold cross-validation (Fig6, graph a). For this, we first compared C-indices by type of input required: (i) a 'base' Cox model including variables defined by pathologist using H&E images alone (histologic subtype, grade and LVSI); (ii) the base model plus tumor stage; (iii) the base model plus tumor stage and molecular EC class. In the cross-validation, given H&E-based input data, the one- and two-arm model discrimination was superior to the base model (C-index = 0.676±0.060). HECTOR model discrimination was superior to the base model plus tumor stage which used the same inputs (C-index = 0.723±0.045), and better or as good as the base model plus tumor stage and molecular EC class (C-index = 0.736±0.033), which requires sequencing and immunohistochemistry.

We further compared HECTOR prognostic value against current prognostic factors in multivariable analysis using HECTOR continuous risk scores as the independent variable. HECTOR retained prognostic value in multivariable models in which known prognostic risk factors (histological subtype, grade, LVSI, tumor stage (2009 FIGO I-III), age, molecular class) were combined as a risk score (referred to as CLINICAL risk score), in which the latter was not prognostic (HECTOR HR = 4.62 (95% Cl: 3.72-5.73; P < 0.005) versus CLINICAL HR = 1.08 (95% Cl: 0.90-1.30; P = 0.402)) (Fig 6, graph b). Similar multivariable analysis including prognostic factors as individual variables showed similar or stronger HECTOR prognostic value (HR = 5.26; 95% Cl: 4.21-6.56; P < 0.005), with only tumor stage III disease retaining statistical significance (HR = 1.50; 95% CI: 1.05-2.14; *P* = 0.026) (Fig. 6, graph c). Other known risk factors were no longer prognostic after inclusion of HECTOR risk score into multivariable models suggesting these factors were captured by HECTOR, including POLEmut and p53abn molecular classes derived from ground-truth sequencing and immunohistochemistry respectively (HR = 0.66 (95% Cl: 0.26-1.69; *P* = 0.384) and HR = 0.90 (95% Cl: 0.61-1.34; *P* = 0.616) respectively) or histological factors such as LVSI (HR: 1.05 95% Cl: 0.77-1.42, P = 0.776).

An unseen external test set containing up to three H&E WSIs from different tissue blocks per patient (n = 151 patients; n = 121 with three WSIs; n = 21 with two and n = 9 with one; Fig. 6 graph e) and follow-up data (2.90-year median follow-up time and 24 events) was collected to assess HECTOR's performance in a real-world diagnostic scenario and to investigate robustness across multiple WSIs derived from the same patient. Performance was first evaluated on this external test set by multitude repetition of blindly selecting one WSI per patient. With this approach, HECTOR achieved a C-index of 0.802±0.013 (Fig. 6, graph f).

Next, we evaluated whether adding extra WSIs per patient improved performance. By random selection of up to two WSIs per patient followed by averaging patient-wise risk scores, the C-index was 0.809±0.007. Selecting up to three WSIs per patient improved the C-index further to 0.813 when using the mean HECTOR risk scores by patient, and 0.816 when using the median (FIG. 6, graph f). The five-year distant recurrence-free survival using the median of HECTOR risk scores per patient was 0.984 (HECTOR low risk, n = 71), 0.748 (HECTOR intermediate risk, n = 44) and 0.556 (HECTOR high risk, n = 37), (P < 0.001) (FIG. 6, graph g). Similar stratification was observed by computing patient-wise mean HECTOR risk scores. A different scenario where the WSIs were merged as one input image was experimented, yielding a C-index decreasing from 0.813 to 0.805. In additional analysis of heterogeneity in predictions between WSIs, 85 cases out of the 142 cases with more than one WSI had consistent HECTOR risk group predictions across the WSIs and only three cases with three WSIs had a different predicted HECTOR risk group for each WSI.

We next examined whether HECTOR could predict treatment benefit using the PORTEC-3 randomized trial in which patients with high-risk localized EC were randomized to adjuvant external beam radiotherapy with or without platinum-based chemotherapy. HECTOR risk scores were predicted on all PORTEC-3 cases (n = 441) which includes the patients who underwent chemotherapy (n = 225); importantly, these 225 cases had not been used in either training or test sets. Analysis of distant recurrence by treatment arm and HECTOR risk score demonstrated a statistically significant interaction between chemotherapy and HECTOR risk score as either continuous or categorical variable (*P*_{INTERACTION} = 0.014 and *P*_{INTERACTION} = 0.064 respectively). We examined this in detail across HECTOR risk groups. Among HECTOR low (n = 92) and HECTOR intermediate risk (n = 177) cases, outcomes were similarly favorable for patients in both treatment arms, as evidenced by similar probability of EC distant recurrence (log-rank P = 0.244 and 0.807 respectively). In contrast, among patients with HECTOR high risk tumors, those who had chemotherapy had significantly improved distant recurrence-free probabilities as compared to patients treated with adjuvant external beam radiotherapy alone (eight-year distant recurrence-free probability of 0.563 versus 0.375; log-rank P = 0.007). Exploratory analysis suggested that the predictive accuracy was greater than that provided by factors currently used to identify patients for chemotherapy, including serous histological subtype, FIGO stage III and the p53abn molecular class.

In summary, HECTOR has showed high accuracy in predicting risk of distant recurrence with a C-index of 0.788 and 0.816 on the internal and external test sets, outperforming gold-standard statistical approaches that require clinicopathological and molecular data manually assessed by pathologists.

It will be appreciated that the above-described embodiments, and their technical features, may be combined with one another in each and every combination, potentially unless there is a conflict between two embodiments or features. That is, each and every combination of two or more of the above-described embodiments is envisaged and included within the present disclosure. One or more features from any embodiment may be incorporated in any other embodiment, and provide a corresponding advantage or advantages.

## Claims

1. A computer-implemented method for predicting a likelihood of an event occurring in a patient having a malignant tumour, the method comprising:
a) inputting, to a trained model, a pathology image of a portion of a malignant tumour of a patient and one or more categorical risk factors associated with the malignant tumour or the patient;
b) extracting, from the pathology image, one or more features relating to one or more properties of the malignant tumour;
c) based on the extracted features and the one or more categorical risk factors, determining, by the trained model, a predicted likelihood of an event occurring in the patient;
d) outputting the predicted likelihood.

2. The computer-implemented method of claim 1, wherein the trained model is further trained to:
predict one or more further categorical risk factors based on the extracted one or more features; and
determine the predicted likelihood of the event based on the extracted features, the one or more input categorical risk factors, and the one or more predicted categorical risk factors.

3. The computer-implemented method of claim 2, wherein the one or more predicted categorical risk factors comprises at least a predicted molecular feature of the malignant tumour, optionally wherein the predicted molecular feature is a predicted molecular classification.

4. The computer-implemented method of any of claim 1 to claim 3, wherein the trained model is further trained to:
treat each categorical risk factor as learnable categorical information; and
form a categorical risk factor embedding for each of the categorical risk factors.

5. The computer-implemented method of claim 4, wherein the trained model is further trained to determine the predicted likelihood by:
forming an image-level embedding for the input pathology image; and
combining each of the categorical risk factor embeddings and the image-level embedding to form a single fused embedding.

6. The computer-implemented method of claim 5, wherein the trained model is further trained to form the single fused embedding by:
applying a gating-based attention mechanism to each of the categorical risk factor embeddings and the image-level embedding; and
performing an outer product to each of the embeddings based on a result of the gating-based attention mechanism.

7. The computer-implemented method of any of claim 1 to claim 6, wherein the one or more input categorical risk factors comprises prognostic metadata or a personal characteristic of the patient, optionally wherein the prognostic metadata comprises a stage category of the malignant tumour, optionally wherein the personal characteristic comprises one or more of an age, ethnicity, body mass index, gender or underlying health condition of the patient.

8. The computer-implemented method of any one of claim 1 to claim 7, wherein the predicted likelihood of an event comprises a predicted time to a clinical endpoint,
optionally wherein the clinical endpoint is a recurrence of the malignant tumour, a progression of the malignant tumour or a death of the patient.

9. A computer-implemented method for training a machine learning model to predict a likelihood of an event occurring in a patient having a malignant tumour, the method comprising:
a) receiving a labelled training set for the machine learning model, the labelled training set comprising, for each of a plurality of patients:
a pathology image of a malignant tumour of the patient;
one or more categorical risk factors associated with the malignant tumour or the patient; and
data relating to the patient's experience with an event; and
b) training the machine learning model, using the labelled training set, to:
extract from each pathology image in the training set, one or more features from the image, and
relate the one or more extracted features and the one or more external categorical risk factors with the event.

10. The computer-implemented method of claim 9, wherein training the machine learning model further comprises:
additionally relating a predicted categorical risk factor to the event, wherein the risk factor is predicted by the machine learning model based on the received pathology image for each of the plurality of patients,
wherein optionally the predicted categorical risk factor is a predicted molecular feature of the malignant tumour, optionally wherein the predicted molecular feature is a predicted molecular classification.

11. The computer-implemented method of claim 9 or 10, wherein the data relating to the patient's experience with the event comprises:
a) a time to the event if the patient has experienced the event; or
b) a time to a most recent assessment for the event if the patient has not experienced the event.

12. The computer-implemented method of claim 9 to claim 11, wherein the event comprises a clinical end point and the predicted likelihood of an occurrence of the event comprises a predicted time to the clinical endpoint,
optionally wherein the clinical endpoint is a recurrence of the malignant tumour, a progression of the malignant tumour or a death of the patient.

13. A trained model obtained from the method of any of any of claim 9 to claim 12.

14. A computer-readable storage device comprising computer-executable instructions, which when executed by a computing system, are capable of causing the computing system to perform the method of any one of any one of claims 1 to 12.

15. A system configured to perform the method of any one of any one of claims 1 to 12.
